# EUROPEAN PATENT APPLICATION

(11) **EP 1 854 786 A1**
(43) Date of publication of application: **14.11.2007**
(21) Application number: 06254590.0
(22) Date of filing: 04.09.2006
(51) Int. Cl.: C07D 213/84, C07D 213/85, C10G 21/06

(54) **Ionic liquids and their use in extraction processes**

(71) Applicant: BP p.l.c., London SW1Y 4PD (GB)
(72) Inventor: Holbrey, John David, Belfast BT9 5AG (GB); Mullan, Claire Lisa, Belfast BT9 5AG (GB)
(74) Representative: Collins, Frances Mary

(57) **Abstract**

There is provided a process for the extraction of at least one aromatic compound from a mixture with at least one aliphatic hydrocarbon, which process comprises contacting said mixture with a salt that is in a liquid state at a temperature below 150°C, said salt having a cation which comprises an aromatic nitrogen-containing heterocyclic ring system, in which a nitrogen atom forming part of said ring system is quaternised and in which said ring system is substituted by at least one electron-withdrawing substituent. Some of said salts are novel.

## Description

This invention relates to ionic liquids and their use. Particularly, it relates to the use of certain ionic liquids for the extraction of aromatic compounds, especially sulfur-containing aromatic compounds, from aliphatic hydrocarbons.

Many oil refinery processes require the separation of aromatic compounds from aliphatic hydrocarbons. For example, it may be desired to reduce the aromatic content of crude oil feeds, while at the other end of the refinery pathway, it may be required to reduce the content of sulfur-containing aromatic compounds in refinery products, for example diesel fuel or gasoline.

Deep desulfurisation of diesel fuels and refinery raffinates has attracted a great deal of attention, due to regulatory requirements to reduce the content of sulfur in fuels. The deep desulfurisation of fuels to achieve very low sulfur contents, for example less than 50 ppm, is a great problem in the production of fuel.

Currently, the separation of sulfur-containing compounds from hydrocarbons in refineries is effected by catalytic hydrogenation. In this process, organic sulfur-containing compounds are converted into hydrogen sulfide and the corresponding hydrocarbons, typically using supported Co-Mo catalysts. However, such processes suffer from major disadvantages: in particular, extreme conditions of temperature and pressure are required, and the ability of current hydrodesulfurisation technologies to fully remove refractory aromatic sulfur compounds, particularly alkylated benzothiophene (BT) and dibenzothiophenes (DBT), is limited. An effective solvent extraction process would offer many advantages, but no such process is currently known which is effective enough to be used commercially.

Room-temperature ionic liquids (*i.e.* ionic materials that are in a liquid state at a temperature of below 150°C, especially at a temperature of below 100°C) have been shown to be of great value in a wide variety of applications. US 7,001,504, Esser et al, Green Chemistry, 2004, 6, 316, and Nie et al, Energy and Fuels, 2006, ASAP Web Release DOI:10.1021/ef06170i, describe the use of certain ionic liquids for deep desulfurisation of oil refinery streams. We have now found a class of ionic liquids which gives improved performance in this process.

Accordingly, the present invention provides a process for the extraction of at least one aromatic compound from a mixture with at least one aliphatic hydrocarbon, which process comprises contacting said mixture with a salt that is in a liquid state at a temperature below 150°C, said salt having a cation which comprises an aromatic nitrogen-containing heterocyclic ring system, in which a nitrogen atom forming part of said ring system is quaternised and in which said ring system is substituted by at least one electron-withdrawing substituent.

Throughout this Specification, the salt used in the present invention will be referred to as an ionic liquid. The salt must be liquid at a temperature of less than 150°C, and is preferably liquid at a temperature of less than 100°C. Most of said salts are novel, and the invention therefore also provides such novel salts *per se.*

The process of the invention finds application in many oil refining processes. It may for example be used to treat any refinery feedstock, for example crude oil, or product, for example a paraffin oil, diesel, gasoline, or lubricating oil. The process is particularly useful for the desulfurisation of refinery products, especially diesel or gasoline, for example as a final stage polishing step. Although any aromatic compound may be extracted from a mixture with one or more aliphatic compounds, preferably the process of the invention is used to extract sulfur-containing aromatic compounds, for example thiophenes, benzothiophenes, dibenzothiophenes and alkyldibenzothiophenes. The process is particularly useful for the extraction of polyaromatic compounds.

The ionic liquids used in the process of the invention are substantially immiscible with aliphatic hydrocarbons. Thus, the process of the invention may be carried out simply by mixing the ionic liquid with the mixture to be treated, to form a two-phase system. Aromatic compounds present in the mixture being treated are preferentially extracted into the ionic liquid phase, an adduct, or complex, being formed between the aromatic compound and the ionic liquid. The ionic liquid phase may then be separated from the hydrocarbon phase by any suitable physical means such as gravity or centrifugal separation. If desired, the ionic liquid may be used as part of an extractive distillation, when the mixture to be treated is mixed with ionic liquid, and the aliphatic hydrocarbon stream is then distilled off, preferably continuously.

If desired, the ionic liquid may be introduced together with a co-solvent. Suitable co-solvents include nitriles, for example acetonitrile, lactones, for example gamma butyrolactone, amides, for example DMF, other nitrogen-containing solvents, for example amines and pyrrolidones, sulfur-containing solvents, for example sulfolane or DMSO, and other oxygen-containing solvents, for example polyethers such as polyethylene glycol and polypropylene glycols, which are miscible with the ionic liquid and substantially immiscible with aliphatic hydrocarbons. Preferably however the ionic liquid is introduced in the absence of any co-solvent.

Once the ionic liquid phase has been separated from the aliphatic hydrocarbon, it may if desired be treated to remove the extracted aromatic compound, and then recycled to the process of the invention. Any suitable method, for example distillation, precipitation or stripping, may be used for separation of the aromatic compound or the aromatic compound/ionic liquid adduct; from the ionic liquid.

The process of the invention may be carried out at any suitable temperature, for example from ambient temperature to 150°C, especially from ambient temperature to 100°C, especially at ambient temperature. Naturally it is usually most economic to carry out the process without the application of heat, and refinery product streams may conveniently be treated at the temperature at which they emerge from the refinery, which is typically up to about 100°C. Suitably the process is carried out at atmospheric pressure, although superatmospheric pressure may be used if desired. The process may be carried out either as a continuous process or as a batchwise process. If desired, the process may be repeated more than once on the same hydrocarbon stream, to provide successive reduction in the content of aromatic compounds.

The cation which is present in the salt used in the present invention comprises an aromatic nitrogen-containing heterocyclic ring system, in which a nitrogen atom forming part of said ring system is quaternised and in which said ring system is substituted by at least one electron-withdrawing group. The ring system suitably contains up to 14 atoms in the ring(s) and may be monocyclic, bicyclic or tricyclic, but is preferably monocyclic or bicyclic, especially monocyclic. Suitable ring systems include for example those derived from pyrrole, oxazole, thiazole, imidazole, pyridine, pyridazine, pyrimidine, pyrazine, triazine, indole, isoindole, indazole, benzimidazole, benzthiazole, purine, quinoline and isoquinoline. Preferred ring systems are those derived from quinoline, imidazole and, especially, pyridine.

In the cation, a nitrogen atom forming part of the ring system is quaternised, the resulting substituent on the nitrogen atom being, for example, hydrogen or an alkyl group having up to 16 carbon atoms optionally substituted by one or more of the same or different substituents selected from phenyl groups and halogen atoms, and optionally interrupted by one or more oxygen, nitrogen and/or sulfur atoms. Preferably the substituent is an unsubstituted alkyl group having up to 16 carbon atoms, preferably from 4 to 12 carbon atoms.

At least one electron-withdrawing substituent (i.e. a substituent which is more electron-withdrawing than a hydrogen atom in the same position) must be present on the ring system of the cation. Suitable substituents include, for example, fluorine and chlorine atoms, and COR, COOR, C₁₋₄ haloalkyl or haloalkoxy (especially fluoro-containing groups, for example CF₃ and OCF₃), SO₃H, NR₃⁺, nitro and cyano groups, in which each R independently represents a hydrogen atom or a C₁₋₄ alkyl group, especially a methyl group. More than one, for example two, three or four, electron withdrawing substituents may be present if desired, and in this case, each substituent may be the same or different. The ring system may also if desired contain one or more, for example one, two or three, substituents in addition to the electron-withdrawing substituent(s), provided that these additional substituents are inert, i.e. do not adversely affect the overall electron-withdrawing effect caused by the electron-withdrawing substituent(s). Suitable inert substituents include alkyl and alkoxy groups having up to 4 carbon atoms, for example methyl or methoxy groups.

The nature of the anion present in the salt used in the present invention is not crucial, provided that the combination of anion and cation results in a salt which is liquid at a temperature of less than 150°C. In general, larger, less symmetrical anions are more likely to result in an ionic liquid than smaller, symmetrical anions. Preferred anions for use in the present invention include alkyl or haloalkyl methanamides (for example tristrifluoromethanesulfonylamide), alkyl or haloalkyl sulfonylamides (for example bistrifluoromethanesulfonylamide, also referred to as bis(trifyl)imide or bistriflamide), alkyl or haloalkyl sulfates (for example methylsulfate and octylsulfate), alkyl or haloalkyl sulfonates (for example methanesulfonate, trifluoromethanesulfonate and dioctylsulfosuccinate), carboxylates (for example methanoate, ethanoate, benzoate and lactate), dicyanamide ([C(CN)₂]⁻), azolates (for example 1,2,4-triazolate), phosphates or phosphonates (for example dimethylphosphate and hexafluorophosphate), perhalides, pseudohalides (for example cyanate and thiocyanide), and various fluorinated anions (for example tetrafluoroborate, perfluoroalkylfluorophosphates, and fluorinated borates).

Salts containing the same cation as those contained in the novel ionic liquids of the present invention are known, for example from Lymar et al, J. Phys Chem. B, 1998, 102, 2811-2819, Jackson and Fung, Mol. Cryst. Lig. Cryst., 1997, 303, 73-78, and Zhu and Kochi, Organometallics 1999, 18, 161. However, most of the known, compounds contain anions which do not render the resulting salt liquid at a temperature of less than 150°C, the exception being 1-methyl-3-cyanopyridinium triflate, disclosed by Zhu and Kochi. Accordingly, the invention provides a salt that is in a liquid state at a temperature below 150°C, said salt having a cation which comprises an aromatic nitrogen-containing heterocyclic ring system, in which a nitrogen atom forming part of said ring system is quaternised and in which said ring system is substituted by at least one electron-withdrawing substituent; provided that said salt is not 1-methyl-3-cyanopyridinium triflate.

Typical ionic liquids which may be used in the present invention include for example:
1-Methyl-4-cyanopyridinium methylsulfate
1-Methyl-4-cyanopyridinium bis(trifyl)amide
1-Ethyl-4-cyanopyridinium ethylsulfate.
1-Ethyl-4-cyanopyridinium bis(trifyl)amide
1-Butyl-4-cyanopyridinium bis(trifyl)amide
1-Hexyl-4-cyanopyridinium bis(trifyl)amide
1-Octyl-4-cyanopyridinium bis(trifyl)amide
1-Octyl-4-cyanopyridinium hexafluorophosphate
1-Dodecyl-4-cyanopyridinium bis(trifyl)amide
1-Hexadecyl-4-cyanopyridinium bis(trifyl)amide
1-Methyl-3-cyanopyridinium methylsulfate
1-Methyl-3-cyanopyridinium bis(trifyl)amide
1-Ethyl-3-cyanopyridinium ethylsulfate
1-Ethyl-3-cyanopyridinium bis(trifyl)amide
1-Butyl-3-cyanopyridinium bis(trifyl)amide
1-Butyl-3-cyanopyridinium hexafluorophosphate
1-Ethyl-2-cyanopyridinium ethylsulfate
1-Ethyl-2-cyanopyridinium bis(trifyl)amide.

The novel compounds of the invention may be prepared by methods analogous to known methods. They may for example be prepared by quatemising an aromatic compound which comprises a nitrogen-containing heterocyclic ring system substituted by at least one electron-withdrawing group, and if desired or required, subsequently replacing the anion in the resulting salt with a different anion. For example, the appropriately substituted heterocycle may be reacted with an alkylating agent, such as an alkyl bromide, followed by metathesis of the anion, to give the required ionic liquid. As a further example, methyl- and ethyl-substituted salts may be prepared by alkylation with dimethylsulfate and diethylsulfate respectively, typically in toluene at around 100°C, following the procedure illustrated by the following scheme for preparing 1-ethyl-4-cyanopyridinium ethylsulfate and 1-ethyl-4-cyanopyridinium bis(trifyl)amide: 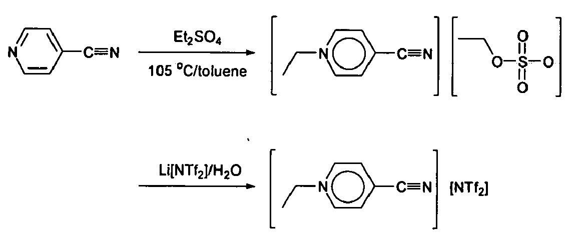 in which NTf₂ represents bis{(trifluoromethyl)sulfonyl}amide (bis(trifyl)amide), [N(SO₂CF₃.)₂], while longer chain analogues may be prepared by alkylation with bromoalkanes in toluene at 135-160°C in sealed vessels. Metathesis of the alkylsulfate or bromide anions to other anions such as bistrifylamide may conveniently be achieved in aqueous solution at room temperature.

The following Examples illustrate the invention.

### Examples 1-19: Synthesis of Novel Ionic Liquids

### Example 1:1-Methyl-4-cyanopyridinium methylsulfate.

A solution of 4-cyanopyridine (30 g, 0.3 mol) and dimethylsulfate (40 cm³) in acetonitrile (100 cm³) was heated with stirring to 45 °C for 24 hours. On cooling to room temperature, the product precipitated as colourless crystals. Melting point 108-115°C.

Example 2. *1-Methyl-4-cyanopyridinium bis(trifyl)amide.* Metathesis of 1-Methyl-4-cyanopyridinium methylsulfate (50g, 0.22 mol) in water with lithium bis(trifyl)amide (62g, 1 eq) resulted in separation of the ionic liquid as a colourless liquid which was collected, washed with water and spontaneously crystallised upon agitation and was collected by filtration, drying in air. Melting point 52 °C. ¹H NMR (dmso-*d₆*) 9.28 (d, 2H) 8.68 (d, 2H),4.43 (s, 3H).

### Example 3: 1-Ethyl-4-cyanopyridinium ethylsulfate.

A solution of 4-cyanopyridine (10.4 g, 0.1 mol) and diethylsulfate (15.4 g, 0.1 mol) in toluene (75 cm³) was heated with stirring at reflux for 6 hours, resulting in the formation of the product as a dense pale yellow liquid phase. The product.was separated from the reaction mixture, washed with toluene (20 cm³) and hexane (30 cm³) then dried under reduced pressure with heating at 70°C and then *in vacuo* to yield a pale yellow viscous liquid. Melting point <25 °C. ¹H NMR (dmso-d₆) 9.38 (2H, d), 9.37 (2H, d), 4.71 (2H, q), 3.73 (2H, q), 1.55 (3H, t), 1.09 (3H, t).

### Example 4: 1-Ethyl-4-cyanopyridinium bis(trifyl)amide.

Metathesis of 1-ethyl-4-cyanopyridinium ethylsulfate (8.00g, 0.03 mol) in water with lithium bis(trifyl)amide (8.83 g, 1 eq) resulted in separation of the ionic liquid as a yellow cloudy liquid phase. The mixture cleared to two transparent liquid phases on heating to 70°C, and the lower yellow ionic liquid was separated, washed with water and dried under reduced pressure and then *in vacuo* with heating to 80°C to yield a clear pale yellow liquid which solidified on standing. The product was immiscible with dichloromethane, and on contact with water changes colour back to an intense yellow as the ionic liquid saturated with water. Mp 33-35°C. ¹H NMR (dmso-d₆) 9.37 (2H, d), 8.68 (2H, d), 4.70 (2H, q), 1.56 (3H, t).

### Example 5: 1-Butyl-4-cyanopyridinium bis(trifyl)amide.

4-Cyanopyridine (20.8 g, 0.2 mol) and 1-bromobutane (42 g, 1.5 eq) were combined and heated with stirring at 135°C in a round-bottomed flask sealed with a Safe-Lab® pressure seal. After heating for 18 h, the resultant yellow solid mass was taken, filtered rapidly to remove excess 1-bromobutane, washed with ethanoate which removed the yellow colouration, and then dried under reduced pressure to yield a pale cream coloured microcrystalline powder. This powder was mixed with water and with Li[NTf_{2]} as described above. The lower ionic liquid phase was separated, extracted into CH₂Cl₂ and dried with heating under reduced pressure to give a clear, pale yellow liquid which was immiscible with CHCl₃ and soluble in dmso. Tg -45°C. ¹H NMR (dmso-*d₆*) 9.35 (2H, d), 8.69 (2H, d), 4.65 (2H, t), 1.90 (3H, tt), 1.292 (2H, tt), 0.90 (3H, t).

### Example 6: 1-Hexyl-4-cyanopyridinium bis(trifyl)amide.

4-Cyanopyridine and 1-bromohexane were heated in toluene at 16-0 °C in a sealed vessel for 24 h. with stirring. On cooling, colourless crystals of 1-hexyl-4-cyanopyridinium bromide were formed and were collected by filtration, washing with diethylether. Water and Li[NTf₂] were added and the product formed as a dense yellow phase liquid which was collected, extracted into dichloromethane dried over anhydrous Na₂SO₄, filtered and dried in vacuo. Melting point <25°C.

### Example 7: 1-Octyl-4-cyanopyridinium bis(trifyl)amide.

4-Cyanopyridine and bromooctane heated at reflux in toluene for 24 h. gave a yellow solution which was cooled and triturated with ethylethanoate giving a cream precipitate from yellow solution. Water and Li[NTf₂] were added to produce a dense brown liquid which was extracted into CH₂Cl₂ and dried *in vacuo.* Melting point <25°C. ¹H NMR (dmso-d₆) 9.35 (2H, d), 8.69 (2H, d), 4.64 (2H, t), 3.29 (0.09H, s, H₂O).1.92 (2H, m), 1.25 (10H, m), 0.84 (3H, t).

### Example 8: 1-Octyl-4-cyanopyridinium hexafluorophosphate.

Prepared by a method analogous to that of Example 7 above using sodium hexafluorophosphate instead of Li[NTf₂], to produce a fine colourless crystalline powder which was collected by filtration and dried in air followed by heating under reduced pressure at 50°C. Melting point 122-124°C. ¹H NMR (dmso-d₆) 9.30 (2H, d), 8.64 (2H, d), 4.62 (2H, t), 3.47 (s, H₂O), 1.90 (2H, m), 1.25 (10H, m) 0.83 (3H, t).

### Example 9: 1-Dodecyl-4-cyanopyridinium bis(trifyl)amide.

Prepared by a method analogous to that of Example 7 above, using 1-bromodecane instead of 1-bromooctane, to produce a fine colourless crystalline powder which was collected by filtration and dried in air followed by heating under reduced pressure at 50°C. Melting point 52°C. ¹H NMR (dmso-*d₆*) 9.342 (2H, d), 8.79 (2H, d), 4.634 (2H, t), 1.909 (2H, m), 1.221 (18H, m), 0.834 (3, t)

### Example 10: 1-Methyl-3-cyanopyridinium methylsulfate.

A solution of 3-cyanopyridine (30 g, 0.3 mol) and dimethylsulfate (40 cm³) in acetonitrile (50 cm³) was heated with stirring to 45°C for 24 hours. The solvent was removed under reduced pressure to give the product as a pale brown liquid. Melting point <25°C.

### Example 11: 1-Methyl-3-cyanopyridinium bis(trifyl)amide.

Metathesis of 1-methyl-3-cyanopyridinium methylsulfate (40.0g, 0.166 mol) in water (100 cm³) with lithium bis(trifyl)amide (47.7g, 0.166 mol) resulted in separation of the ionic liquid as a colourless liquid which was collected, washed with water and dried under vacuum to yield the product as a colourless solid. Melting point 63-65°C, ¹H NMR (dmso-*d₆*) 9.73 (s, 1H), 9.24 (d, 1H), 9.06 (d, 1H), 8.33 (dd, 1H) 4.38 (s, 3H).

### Example 12: 1-Ethyl-3-cyanopyridinium ethylsulfate.

Diethylsulfate (154 g, 1 mol) was added dropwise to a solution of 3-cyanopyridine (104g, 1 mol) in toluene (250 cm³) heated to 100°C with stirring. After addition, the reaction mixture was heated for a further 4 hours, then cooled to room temperature. The product formed as a dense pale yellow liquid which was separated, washed with toluene and then with hexane, dried under reduced pressure and finally in vacuo to yield a pale yellow viscous liquid. Melting point <25°C. ¹H NMR (dmso-d₆) 9.774 (1H, s, C(2)-H), 9.330 (1H, d, C(6)-H), 9.066 (1H, d, C(4)-H), 8.335 (1H, dd, C(5)-H), 4.659 (2H, q), 3.728 (2H, q), 1.556 (3H, t), 1.087 (3H, t).

### Example 13: 1-Ethyl-3-cyanopyridinium bis(trifyl)amide.

Metathesis of 1-ethyl-3-cyanopyridinium ethylsulfate (95.0g) in water (200 cm³) with lithium bis(trifyl)amide (110g) in water (100 cm³) resulted in separation of the product as a colourless crystalline solid which was collected by filtration, washed with water and dried under reduced pressure. Melting point 73 °C. ¹H NMR (dmso-d₆) 9.782 (1H, s, C(2)-H), 9.321 (1H, d, C(6)-H, 6.12), 9.057 (1H, d, C(4)-H, 8.12), 8.334 (1H, dd, C(5)-H, 7.9, 6.4), 4.645 (2H, q, 7.324), 3.426 (2H, q, 7.2), 1.557 (3H, t, 7.33)

### Example 14: 1-Butyl-3-cyanopyridinium bis(trifyl)amide.

A solution of lithium bis(trifyl)amide (19.0g) in water (50 cm³) was added to a solution of 1-butyl-3-cyanopyridinium bromide (16.82 g) in water (50 cm³). The resultant cloudy mixture was heated to 60°C forming two separate phases. The upper aqueous phase was decanted and the dense product phase was collected, washed with water and dried under vacuum to yield the product as a pale brown liquid. Melting point <25°C. ¹H NMR (dmso-d₆) 9.786 (1H, s, C(2)-H), 9.315 (1H, d, C(6)-H, J1 6.06), 9.068 (1H, d, C(4)-H, 8.13), 8.335 (1H, dd, C(3)-H, J1 6.06, 8.13), 4.609 (2H, t, J1 7.5), 1.925 (2H, m), 1.305 (2H, m), 0.910 (3H, t, J1 7.35).

### Example 15: 1-Bulyl-3-cyanopyridinium hexafluorophosphate.

Na[PF₆] (0.7g) was added to a solution of 1-butyl-3-cyanopyridinium bromide (0.9 g) in water (10 cm³). The product precipitated from the mixture as colourless crystals which were collected by filtration and air dried. Melting point 110-111°C.

### Example 16: 1-Methyl-2-cyanopyridinium methylsulfate.

A solution of 3-cyanopyridine (30 g, 0.3 mol) and dimethylsulfate (40 cm³) in acetonitrile (50 cm³) was heated with stirring to 45°C for 24 hours. The solvent was removed under reduced pressure to give the product as a crystalline solid.

### Example 17: 1-Methyl-2-cyanopyridinium bis(trifyl)amide.

Metathesis of 1-methyl-2-cyanopyridinium methylsulfate (40.0g, 0.166 mol) in water (100 cm³) with lithium bis(trifyl)amide (47.7g, 0.166 mol) resulted in separation of the ionic liquid as a colourless liquid which was collected, washed with water and dried under vacuum to yield the product as an oil. Melting point <25°C. ¹H NMR (dmso-d₆) 9.39 (d, 1H), 8.84 (dd, 1H), 8.81 (dd, 1H), 8.46 (d, 1H), 4.54 (s, 3H).

### Example 18: 1-Ethyl-2-cyanopyridinium ethylsulfate.

Diethylsulfate (50 g, 0.3 mol) was added dropwise to a solution of 2-cyanopyridine (25g, 0.25 mol) in toluene (70 cm³) heated to 100°C with stirring. After addition, the reaction mixture was heated for a further 4 hours, then cooled to room temperature. The product formed as a dense pale yellow liquid which was separated, washed with toluene and then with hexane, dried under reduced pressure and finally in vacuo to yield a pale yellow solid. Melting point 34-36°C. ¹H NMR (dmso-d₆) 9.429 (1H, d, C(6)-H, 6.024), 8.81 (2H, m, C(3)-H and C(5)-H), 8.461 (1H, dd, 7), 4.834 (2H, q, 7.3), 3.719 (2H, q, 7.1), 1.620 (3H, t, 7.3), 1.086 (3H, t, 7.12).

### Example 19: 1-Ethyl-2-cyanopyridinium bis(trifyl)amide.

Metathesis of 1-ethyl-2-cyanopyridinium ethylsulfate (21.8 g, 0.086 mol) in water (100 cm³) with lithium bis(trifyl)amide (24.7 g) in water (100 cm³) resulted in separation of the product as a colourless liquid which was washed with water and dried under reduced pressure to yield a colourless crystalline solid. Melting point 36-38°C. ¹H NMR (dmso-d₆) 9.429 (1H, d, C(6)-H, 6.024), 8.81 (2H, m, C(3)-H and C(5)-H), 8.461 (1H, dd, 7), 4.834 (2H, q, 7.3), 1.086 (3H, t, 7.12).

### Example 20: Extraction of dibenzothiophene from dodecane

This Example illustrates the extraction of dibenzothiophene (DBT), which is a representative refractory sulfur compound known to be difficult to desulfurise by the classical dehydrogenation method.

The ionic liquids 1-butyl-4-cyanopyridinium bis(trifyl)amide and 1-hexyl-4-cyanopyridinium bis(trifyl)amide were screened with dibenzothiophene -containing dodecane added at 0.0163 or 0.0033 moles per litre, corresponding to approximately 500 and 100 ppm sulfur respectively. Samples of the ionic liquid and oil phase were intensively stirred with a magnetic stirrer at a volume ratio of 1:4 ionic liquid:oil for 60 minutes in a thermostatted vessel, then stood for 15 minutes at temperature to ensure equilibration and separation of the two phases. The oil was separated from the two-phase mixture, and the dibenzothiophene content was determined by GC/MS analysis. The results are given in Table 1.

**Table 1**

| IL extracting phase | initial sulfur content (as DBT) /ppm | Sulfur content after treatment /ppm (percentage extracted in parentheses) | | | |
|---|---|---|---|---|---|
| | | Temperature | | | |
| | | 40 °C | 50 °C | 60 °C | 70 °C |
| 1-butyl-4-cyanopyridinium bis(trifyl)amide | 521 | 359 (31) | 358 (31) | 378 (27) | 428 (18) |
| | 104 | 68 (35) | 69 (34) | 73 (30) | 80 (23) |
| 1-hexyl-4-cyanopyridinium bis(trifyl)amide | 521 | 328 (37) | 327 (37) | 356 (32) | 366 (30) |
| | 106 | 63 (39) | 62 (41) | 69 (34) | 69 (33) |

The results show a good percentage of the DBT being extracted in a single pass.

### Example 21: Extraction of dibenzothiophene from kerosene.

Samples of kerosene spiked with dibenzothiophene (total sulfur content as dibenzothiophene of 609, 1409, and 7391 ppm) were contacted with 1-ethyl-4-cyanopyridinium bis(trifyl)amide ionic liquid at a 1:10 volume ratio (1 cm³ ionic liquid and 10 cm³ kerosene) at 60°C, and efficiently mixed with a homogeniser (UltraTurrax) for 30 sec. The samples were then allowed to settle for 5 min at 60°C. A sample of the lower ionic liquid phase was taken (50 µL diluted to 1.5 mL with acetonitrile) and analysed by reverse phase hplc. Dibenzothiophene was identified in the ionic liquid phase, and the concentration was calculated by comparison with standard calibration samples. The results are shown in Table 2.

**Table 2**

| Initial sulfur content, as DBT/ppm | sulfur content after treatment/ppm (percentage extracted in parentheses) |
|---|---|
| 7391 | 6858 (7) |
| 1409 | 1261 (11) |
| 609 | 538 (12) |

Significant sulfur extraction is seen. The extraction efficiency appears to increase with decreasing sulfur content in the feed and indicates that using a multistage extraction process, ultradeep desulfurisation of refractory sulfur-containing aromatics from hydrocarbons could be achieved.

### Example 22: Extraction of 1-methylnaphthalene from dodecane.

Three ionic liquids, 1-methyl-4-cyanopyridinum bis(trifyl)amide, 1-ethyl-4-cyanopyridinum bis(trifyl)amide, and 1-butyl-4-cyanopyridinum bis(trifyl)amide, were screened against a model oil comprising dodecane containing 0.0185 moles per litre of 1-methylnaphthalene. Preheated samples of the ionic liquid and oil phase were intensively stirred in a thermostated high-shear mixer at volume ratios of either 1:9 or 1:3 ionic liquid:oil for 2.5 minutes at 70°C, then equilibrated in heated vials at 70°C for 1 hour. The model oil was separated from the two-phase mixture and the 1-methylnaphthalene content of the hydrocarbon phase was determined by GC/MS analysis. The results are shown in Table 3.

**Table 3**

| IL extracting phase | contact ratio | initial concentration/ mol L⁻¹ | concentration after treatment/mol L⁻¹ |
|---|---|---|---|
| 1-methyl-4-cyanopyridinium bis(trifyl)amide | 1:9 | 0.018 | 0.016 |
| 1-ethyl-4-cyanopyridinium bis(trifyl)amide | 1:3 | 0.018 | 0.015 |
| 1-butyl-4-cyanopyridinium bis(trifyl)amide | 1:3 | 0.018 . | 0.015 |

### Example 23: Extraction of dimethyldibenzothiophene from dodecane

Extraction of dimethyldibenzothiophene (DMDBT) from dodecane was investigated using three 1-alkyl-4-cyanopyridinium bis(trifyl)amide ionic liquids (alkyl = ethyl, butyl, and hexyl) as extractants. Equal volumes of spiked dodecane (containing either 500 or 250 ppm sulfur) and ionic liquid were contacted and stirred vigorously for 4 hours at 50 °C to ensure equilibration. Stirring was stopped and the two phases separated under gravity.

The concentration of dimethyldibenzothiophene in the upper, dodecane phase was determined by GC/MS, comparing with calibration standards and the initial spiked dodecane solutions. The results are shown in Table 4.

**Table 4**

| IL extracting phase | initial sulfur content (as DMDBT)/ppm | sulfur content after treating/ppm (percent extracted in parentheses) | |
|---|---|---|---|
| | | Temperature (°C) | |
| | | 50 | 60 |
| 1-ethyl-4-cyanopyridinium bis(trifyl)amide | 250 | 175 30) | 179 (28) |
| | 500 | 354(29) | 368(26) |
| 1-butyl-4-cyanopyridinium bis(trifyl)amide | 250 | 141 (44) | 142 (43) |
| | 500 | 284 (43) | 289 (42) |
| 1-hexyl-4-cyanopyridinium bis(trifyl)amide | 250 | 117 (53) | 120 (52) |
| | 500 | 227 (55) | 241 (52) |

### Example 24: Extraction of dibenzothiophene from Harding crude oil.

In this experiment, dibenzothiophene was added as a spike to Harding crude oil at a concentration corresponding to 510ppm of sulfur, and extracted using the ionic liquid 1-butyl-4-cyanopyridinium bis(trifyl)amide at 50°C using a 1:1 contacting volume ratio, using a process analogous to that described in the previous examples. The results are given in Table 5.

**Table 5**

| Initial sulfur content (as DBT)/ppm | Sulfur content (DBT) after treatment/ppm |
|---|---|
| 510 | 259 |

## Claims

1. A process for the extraction of at least one aromatic compound from a mixture with at least one aliphatic hydrocarbon, which process comprises contacting said mixture with a salt that is in a liquid state at a temperature below 150°C, said salt having a cation which comprises an aromatic nitrogen-containing heterocyclic ring system, in which a nitrogen atom forming part of said ring system is quaternised and in which said ring system is substituted by at least one electron-withdrawing substituent.

2. A process as claimed in claim 1, which is applied as a treatment for a refinery feedstock or a refinery product.

3. A process as claimed in claim 2, which is applied as a desulfurisation process for a refinery product.

4. A process as claimed in any one of claims 1 to 3, which is carried out at a temperature of from ambient temperature to 150°C.

5. A process as claimed in claim 4, which is carried out on a stream exiting a refinery, and which is carried out at the temperature at which said stream emerges from the refinery.

6. A process as claimed in any one of the preceding claims, in which said salt is in a liquid state at a temperature below 100°C.

7. A process as claimed in any one of the preceding claims, in which said ring system contains up to 14 atoms in the ring(s).

8. A process as claimed in any one of the preceding claims, in which said ring system is monocyclic or bicyclic

9. A process as claimed in claim 8, in which said ring system is derived from pyrrole, oxazole, thiazole, imidazole, pyridine, pyridazine, pyrimidine, pyrazine, triazine, indole, isoindole, indazole, benzimidazole, benzthiazole, purine, quinoline and isoquinoline.

10. A process as claimed in claim 9, in which said ring system is derived from quinoline, imidazole or pyridine.

11. A process as claimed in any one of the preceding claims, in which, in the cation present in said salt, the quaternised nitrogen atom is substituted by hydrogen or an alkyl group having up to 16 carbon atoms optionally substituted by one or more of the same or different substituents selected from phenyl groups and halogen atoms, and optionally interrupted by one or more oxygen, nitrogen and/or sulfur atoms.

12. A process as claimed in claim 11, in which said quaternised nitrogen atom is substituted by an unsubstituted alkyl group having up to 16 carbon atoms.

13. A process as claimed in any one of the preceding claims, in which said electron-withdrawing substituent is selected from fluorine and chlorine atoms, and COR, COOR, C₁₋₄ haloalkyl or haloalkoxy, SO₃H, NR₃⁺, nitro and cyano groups, in which each R independently represents a hydrogen atom or a C₁₋₄ alkyl group.

14. A process as claimed in any one of the preceding claims, in which the anion in said salt is selected from alkyl or haloalkyl methanamides, alkyl or haloalkyl sulfonylamides, alkyl or haloalkyl sulfates, alkyl or haloalkyl sulfonates, carboxylates, dicyanamide, azolates, phosphates, phosphonates, pseudohalides, and fluorinated anions.

15. A process as claimed in any one of claims 1 to 5, in which said salt is:
1-Methyl-4-cyanopyridinium methylsulfate,
1-Methyl-4-cyanopyridinium bis(trifyl)amide,
1-Ethyl-4-cyanopyridinium ethylsulfate,
1-Ethyl-4-cyanopyridinium bis(trifyl)amide,
1-Butyl-4-cyanopyridinium bis(trifyl)amide,
1-Hexyl-4-cyanopyridinium bis(trifyl)amide,
1-Octyl-4-cyanopyridinium bis(trifyl)amide,
1-Octyl-4-cyanopyridinium hexafluorophosphate,
1-Dodecyl-4-cyanopyridinium bis(trifyl)amide,
1-Hexadecyl-4-cyanopyridinium bis(trifyl)amide,
1-Methyl-3-cyanopyridinium methylsulfate,
1-Methyl-3-cyanopyridinium bis(trifyl)amide,
1-Ethyl-3-cyanopyridinium ethylsulfate,
1-Ethyl-3-cyanopyridinium bis(trifyl)amide,
1-Butyl-3-cyanopyridinium bis(trifyl)amide,
1-Butyl-3-cyanopyridinium hexafluorophosphate,
1-Ethyl-2-cyanopyridinium ethylsulfate, or
1-Ethyl-2-cyanopyridinium bis(trifyl)amide.

16. A salt that is in a liquid state at a temperature below 150°C, said salt having a cation which comprises an aromatic nitrogen-containing heterocyclic ring system, in which a nitrogen atom forming part of said ring system is quaternised and in which said ring system is substituted by at least one electron-withdrawing substituent; provided that said salt is not 1-methyl-3-cyanopyridinium triflate.

17. A salt as claimed in claim 16, which is as defined in any one of claims 6 to 14.

18. A process for the preparation of a salt as claimed in either claim 16 or claim 17, which comprises quatemising an aromatic compound which comprises a nitrogen-containing heterocyclic ring system substituted by at least one electron-withdrawing group, and if desired or required, subsequently replacing the anion in the resulting salt with a different anion.
